# DEMANDE DE BREVET EUROPEEN

(11) **EP 0 611 223 A1**
(43) Date de publication de la demande: **17.08.1994**
(21) Numéro de dépôt: 94420006.2
(22) Date de dépôt: 10.01.1994
(51) Int. Cl.: A61M 39/00

(54) **Dispositif de raccordement entre un récipient et une conduite de passage pour liquides à usage médical**

(30) Priorité: 11.01.1993 IT TO930009
(71) Demandeur: HOSPAL AG, CH-4008 Basel (CH)
(72) Inventeur: Neri, Roberto, I-41037 Mirandola (MO) (IT)

(57) **Abrégé**

Le dispositif (1) comprend une membrane (15) supportée par un corps tubulaire (13) en matière élastomère lequel est relié élastiquement à un corps de support (16) d'un premier raccord (8). Le corps (16) présente un manchon (17) muni d'un filetage (18) et une partie tubulaire (19) pour la fixation à une poche de liquide à usage médical. Le dispositif comprend, en outre, un perforateur (56) supporté par un second raccord (10) fixé à une conduite. Le perforateur (56) est muni d'une pointe (57) et de deux ouvertures (61) et est solidaire d'un manchon (52) ayant un filetage (53) complémentaire de celui du manchon (17) du premier raccord (8). Normalement chaque raccord (8, 10) est fermé par un bouchon qui garantit la stérilité. Au moment de l'utilisation, on enlève les deux bouchons et on visse les deux manchons (17, 52) afin de perforer la membrane (15) au moyen de la pointe (57) du perforateur (56).

## Description

La présente invention concerne un dispositif de raccordement entre un récipient et une conduite de passage pour liquides à usage médical.

Dans les dispositifs de raccordement connus, la conduite est normalement fermée par une membrane protégée par un bouchon d'obturation métallique et est susceptible d'être perforée par un élément de perforation raccordé, par une tubulure, à un dialyseur. La membrane, en général en caoutchouc, présente une certaine épaisseur pour éviter les perforations accidentelles.

Ces dispositifs de raccordement présentent quelques inconvénients. Avant tout, l'épaisseur du caoutchouc demande l'application d'un certain effort pour effectuer la perforation, ce qui implique un risque de blessure accidentelle pour l'opérateur. De plus, pendant la perforation ou à la suite d'une sollicitation du raccord d'accouplement, il peut se produire une fuite de liquide étant donné qu'il n'existe pas de jonction stable entre les éléments. De manière analogue, les dispositifs n'assurent pas l'étanchéité du raccordement en cas de rupture accidentelle par arrachement. Il s'en suit que les dispositifs connus n'assurent pas toujours la stérilité nécessaire.

La présente invention a pour objet de réaliser un dispositif de raccordement de ce type, d'une simplicité maximale et dont la fiabilité permet d'éliminer les inconvénients cités plus haut des connecteurs connus.

Ce but est atteint par le dispositif de raccordement entre un récipient et une conduite de passage pour liquides à usage médical, comprenant une membrane et un élément perforateur relié à une conduite et à un récipient, caractérisé en ce que ladite membrane et ledit élément perforateur sont supportés, respectivement, par un premier et un deuxième raccord, lesdits raccords étant pourvus de moyens de raccordement réciproque, solidaires univoquement entre eux, pour le raccordement stable dudit récipient et de ladite conduite.

Pour permettre une meilleure compréhension de l'invention, il va être décrit une forme de réalisation préférée, illustrée sur les dessins annexés, dans lesquels:
Fig.1 représente une coupe longitudinale d'une partie du dispositif de raccordement selon l'invention;
Fig.2 représente une coupe longitudinale de l'autre partie du dispositif de raccordement selon l'invention;
Fig.3 est une vue de face, en coupe partielle, d'un détail de la Fig.2, à échelle agrandie;
Fig.4 illustre une vue schématique partielle d'une conduite de perfusion;
Fig.5 représente une vue en coupe transversale du dispositif de raccordement dans une étape intermédiaire de raccordement des deux parties du présent dispositif de raccordement; et
Fig.6 représente le dispositif de raccordement à la fin de l'étape de raccordement.

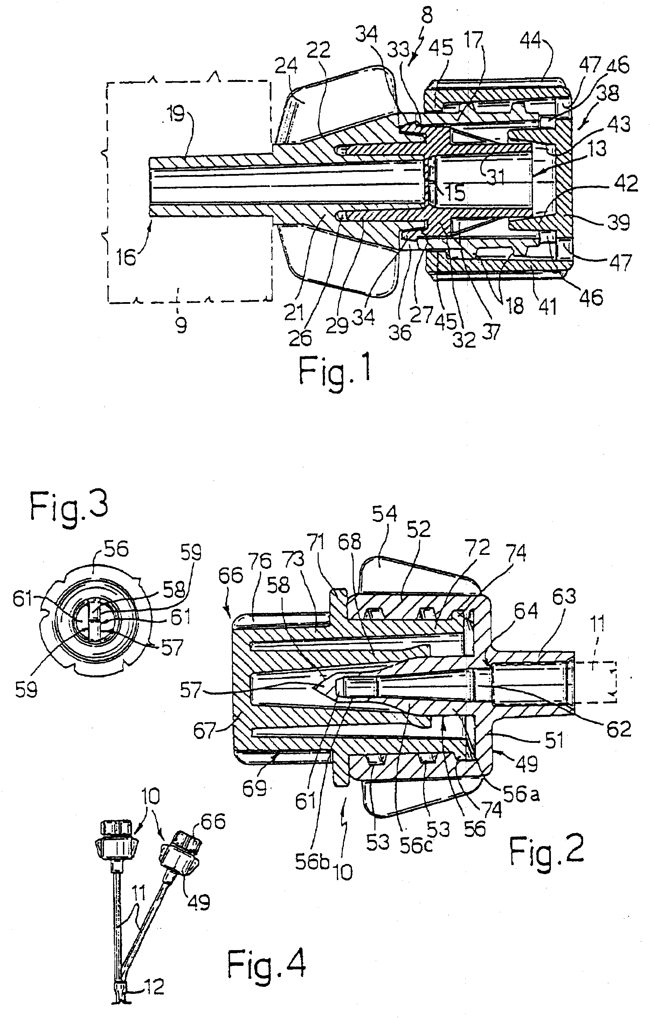

En se référant à la Fig.6, le dispositif de raccordement est désigné, d'une manière générale, par le repère 1. Le dispositif 1 comprend un premier et un deuxième élément de raccordement, désignés par la suite simplement par le premier raccord 8 et le deuxième raccord 10. Le premier raccord 8 est prévu pour être fixé sur un récipient 9 de liquide à usage médical (Fig.1). En particulier, le récipient 9 peut être constitué par une poche souple transparente en matière plastique, par exemple en PVC. Le liquide qu'elle contient peut être constitué par une solution de dialyse.

Le deuxième raccord 10 (Fig.2) est destiné à être fixé à une conduite 11 d'un tubage 12 (Fig.4) relié à un appareillage médical, non représenté. Dans le cas décrit, le tubage définit la tuyauterie d'alimentation de liquide dialysant à un dialyseur et sera désigné par la suite sous le nom de conduit 12. En particulier, le conduit 12 peut être pourvu d'une pluralité de conduites 11, communiquant éventuellement entre elles, chacune d'elles étant munies du raccord 10.

Le raccord 8 (Fig.1) comprend un corps tubulaire 13 en matériau élastomère, par exemple en PVC souple, pourvu d'une membrane de fermeture 15. Le raccord 8 comprend, en outre, un corps de support 16 de corps tubulaire 13, lequel corps 16 étant réalisé par estampage à partir d'un matériau plastique relativement rigide, par exemple du polycarbonate.

Le corps de support 16 est constitué par un manchon 17 muni d'un filetage extérieur 18, d'une partie tubulaire 19, susceptible d'être soudée à la poche 9, et d'une partie intermédiaire 21 entre le manchon 17 et la partie 19.

La partie intermédiaire 21 présente une surface cylindrique interne, dans l'alignement de la partie tubulaire 19, et une surface extérieure 22, sensiblement conique. Il part de la surface 22 deux ailettes 24, diamétralement opposées, aptes à faciliter le raccordement des deux éléments du dispositif de raccordement, comme on le verra de façon plus détaillée par la suite. La partie intermédiaire 21 est pourvue intérieurement d'une première portée 26, de forme cylindrique, et d'une deuxième portée 27, également cylindrique, disposée coaxialement et à l'extérieur de la première portée 26, la profondeur de la première portée 26, dans la direction axiale, étant supérieure à celle de la deuxième portée 27.

Le corps tubulaire 13 est aussi réalisé monobloc et comprend deux parties cylindriques 29 et 31, contiguës entre elles et séparées par la membrane 15. La partie 29 est susceptible de venir se loger dans la portée 26 de la partie 21 et présente une longueur approximativement égale ou légèrement inférieure à la profondeur de la portée 26. Extérieurement, en regard de la membrane 15, le corps tubulaire 13 est pourvu d'une bride 32 à partir de laquelle part un élément annulaire 33, coaxial audit corps tubulaire 13. L'élément annulaire 33 est susceptible de venir en prise dans la portée annulaire 27 et d'être bloqué en position grâce à la présence de deux ergots 34, dirigés en saillie vers l'extérieur, à partir de deux points diamétralement opposés de l'élément annulaire 33 et susceptibles de venir s'enclencher par déclic dans deux fentes correspondantes 36, prévues dans la partie 21.

La bride 32 est, en outre, reliée à la partie 31 du corps tubulaire 13 par l'intermédiaire de deux nervures 37 de raidissement et de guidage axial.

Le raccord 8 est normalement fermé par un bouchon 38 comprenant une paroi de fond 39 d'où part un manchon extérieur 41 et une saillie annulaire interne 42, coaxiale au manchon 41. La saillie annulaire interne 42 comprend une surface intérieure 43 qui va en se rétrécissant en forme de cône vers la paroi de fond 39 et susceptible de coopérer avec la partie 31 du corps tubulaire 13 dans le but de centrer ce dernier par rapport au bouchon 38. La surface extérieure de la saillie annulaire 42 est, par contre, cylindrique et forme avec la surface intérieure du manchon 41 une portée pour l'extrémité du manchon 17 vissée dans le corps de support 16.

Des nervures axiales 44 sont ménagées sur la paroi extérieure du manchon 41 pour améliorer la prise de serrage à la main du bouchon 38. Sur le bord du manchon 41, il est prévu deux ergots 45 diamétralement opposés et dirigés radialement vers l'intérieur. Les ergots 45 sont prévus pour venir en prise dans le filetage 18 du manchon 17 du corps de support 16.

La paroi de fond 39 du bouchon 38 est munie d'une paire de saillies 46 contre lesquelles vient porter le bord du manchon 17 lorsque le vis sage est terminé, et deux ouvertures 47 venues d'estampage. La vapeur de stérilisation du raccord 8 peut pénétrer à travers ces ouvertures 47, ainsi que par le bord du manchon 41 du bouchon 38, jusqu'à la membrane 15. La présence des ouvertures 47 n'affecte pas ultérieurement l'état stérile de la surface extérieure de la membrane 15 étant donné que les portées d'accouplement entre le bouchon 38 et la partie 31 du corps tubulaire 13 forment un labyrinthe infranchissable pour les bactéries qui, comme on le sait, ne se déplacent qu'en ligne droite.

Lors de la confection de la poche de liquide 9, on remplit tout d'abord la poche 9 de liquide à travers une ouverture ménagée sur sa périphérie, après quoi la partie tubulaire 19 du raccord 8 est introduite dans cette ouverture. Ensuite, on procède à la stérilisation de l'ensemble complet 8 et 9, à la vapeur à 120°C. Au cours de cette étape, la partie cylindrique 29 du corps tubulaire 13 se dilate et vient adhérer parfaitement sur la paroi de la portée 26 et assure la stérilité ultérieure de la membrane 15, même après une période de stockage de longue durée du raccord 8. De manière analogue, il existe une parfaite adhérence entre la partie tubulaire 19 et la poche 9, ce qui garantit la stérilité de l'ensemble du groupe formant la poche 8, 9.

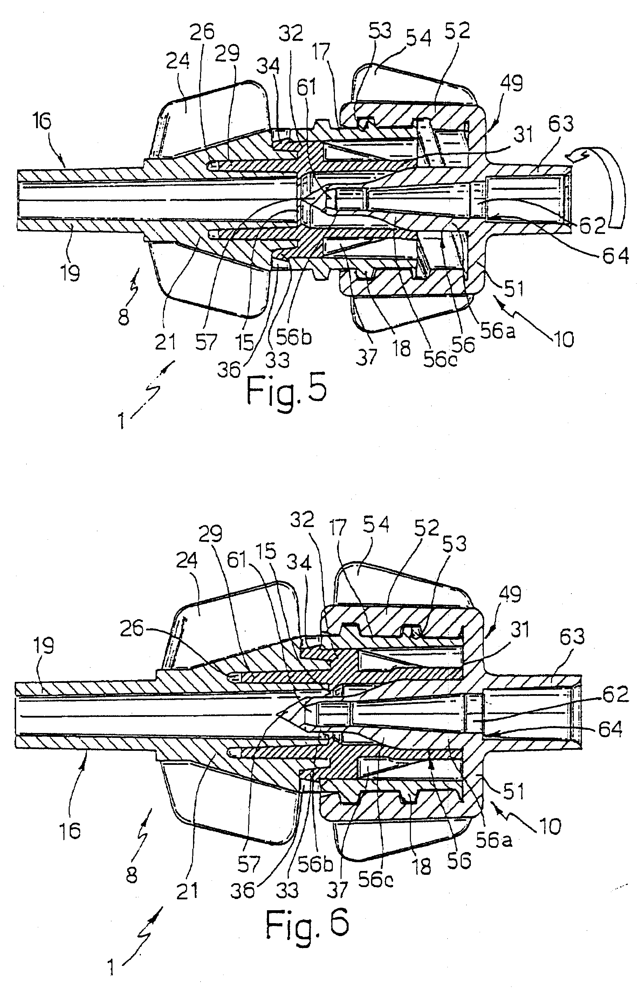

Le raccord 10 de la conduite 11 (Fig.2) comprend un élément de perforation ou perforateur 56, constitué par un élément réalisé monobloc avec le corps de support 49, de préférence en PVC. Le corps de support 49 comprend un collet 51 à partir duquel part, du bord extérieur, un manchon extérieur 52 muni d'un filetage intérieur 53, complémentaire du filetage 18 du manchon 17 (également représenté sur les Fig.5 et 6). Le manchon 52 est muni de deux ailettes 54, diamétralement opposées, pour faciliter la prise du raccord 10 comme détaillé par la suite.

Le perforateur 56 présente une forme tubulaire se terminant par une pointe conique 57 s'étendant sur le diamètre du perforateur 56. La pointe 57 (voir également la Fig.4) présente une forme triangulaire plate, définie par deux parois convergentes 58 et par deux parois parallèles 59 et se raccorde à la partie tubulaire 56, de manière à former deux ouvertures 61 diamétralement opposées, pour mettre en communication l'intérieur du perforateur 56 avec l'extérieur et obtenir un débit élevé, atteignant jusqu'à environ 40 litres/heure.

Le perforateur 56 est constitué par une première partie 56a, partant du collet 51, et d'un diamètre extérieur supérieur au diamètre intérieur de la partie 31 du corps tubulaire 13, pour des raisons qui seront expliquées par la suite; une partie finale 56b, contiguë à la pointe 57 et d'un diamètre extérieur inférieur à celui de la première partie 56 et du diamètre intérieur de la partie 31; ainsi qu'une partie de raccordement 56c, d'un diamètre allant en décroissant à partir de la première partie 56a, jusqu'à la partie finale 56b. Coaxialement au perforateur 56, à partir de la partie opposée au collet 51, s'étend une partie tubulaire extérieure 63, dont l'intérieur est en communication avec l'intérieur du perforateur 56 par l'intermédiaire d'une ouverture centrale 62 du collet 51. La surface intérieure de la partie 63 forme avec le collet 51 un épaulement 64 contre lequel vient porter l'extrémité de la conduite 11 destinée à être soudée à l'intérieur de la partie 63, d'une façon connue en soi.

Le raccord 10 est normalement fermé par un bouchon correspondant, désigné par 66, qui peut être estampé, réalisé de préférence à partir du même matériau que le raccord 10. Le bouchon 66 comprend une paroi de fond 67 d'où partent un manchon intérieur 68 et un manchon extérieur 69, coaxiaux entre eux. Le manchon intérieur 68 possède une paroi latérale intérieure légèrement rétrécie, dont le diamètre maximal est inférieur au diamètre extérieur de la première partie 56a du perforateur 56, pour les raisons qui seront expliquées plus loin.

Le manchon extérieur 69 comprend un épaulement ou collet 71 qui sépare une partie intérieure 72, destinée à être vissée à l'intérieur du manchon 52 du raccord 10 d'une partie extérieure 73 du manchon 69. La partie intérieure 72 est munie de deux ergots 74 dirigés radialement vers l'extérieur, diamétralement opposés l'un par rapport à l'autre, et aptes à venir en prise dans le filetage 53 du manchon 52. La partie extérieure 73 présente une surface extérieure dentelée, de manière à faciliter le vissage et le dévissage du bouchon 66 du raccord 10. Dans le bouchon 66 représenté sur les dessins, cette surface est constituée par quatre secteurs concaves 76, de manière que chaque paire de secteurs opposés 76 puisse venir en prise sur deux des autres.

La longueur réciproque du manchon intérieur 68 du bouchon 66 et des parties 56a - 56c du perforateur 56 est prévue de manière que lorsque le bouchon 66 est vissé sur le corps de support 49, la partie de raccord 56c vient en prise sur le bord du manchon intérieur 68 de manière à provoquer sa dilatation, comme illustré sur la fig. 2. En vissant à fond le bouchon 66, le collet 71 vient en appui contre le bord du manchon 52, tandis que la déformation élastique du manchon 68 ferme hermétiquement la partie finale 56b et la pointe 57 du perforateur 56, et empêche un dévissage accidentel du bouchon 66 hors du manchon 52.

Le groupe composé par le conduit 12 et les raccords 10 est, en général, stérilisé avant son utilisation à l'oxyde d'éthylène.

On procède de la façon suivante pour relier mutuellement les deux raccords 8 et 10.

Tout d'abord, on enlève le bouchon 38 du corps de support 16 du raccord 8 et le bouchon 66 du corps de support 49 du raccord 10, correspondant à la conduite 11. Ensuite, on vis se réciproquement les deux raccords 8 et 10, en engageant réciproquement en prise les filetages complémentaires 18 et 53. Au cours du vissage, grâce à la longueur relative de la partie cylindrique 31 du corps tubulaire 13, on engage tout d'abord la partie intermédiaire 56c du perforateur 56 en prise sur le bord de la partie cylindrique 31 en la dilatant, de manière que la pointe 57 vienne en contact avec la membrane 15, comme indiqué sur la Fig.5.

En continuant de visser les deux filetages 18 et 53, la pointe 57 perfore tout d'abord la membrane 15, puis pénètre ensuite à l'intérieur de la partie cylindrique 29 du corps tubulaire 13, comme indiqué sur la Fig.6. A ce moment, le liquide de la poche 9, traversant le corps de support 16, pénètre dans les ouvertures 61 et, en traversant la partie tubulaire 56 du corps 49, passe dans la conduite 11 du conduit 12.

Il ressort de ce qui précède que les avantages du dispositif de raccordement de l'invention, comparativement aux dispositifs connus, sont évidents. En particulier, grâce à la présence de moyens de raccordement spécifiques entre les deux raccords 8, 10, le raccordement de ces derniers est stable et ne risque pas de se desserrer. En outre, le raccordement et la perforation de la membrane 15 sont simplifiés étant donné qu'ils ne demandent qu'un vissage réciproque entre les raccords 8, 10. De plus, l'interférence entre le bord du manchon intérieur 31 et le perforateur 56 débute même avant la perforation de la membrane 13, ce qui garantit le maintien de la stérilité et l'amorce de passage du liquide par suite de la perforation, qui se produit sous des conditions hermétiques, se fait sans risque de fuites de liquide.

La présence de deux ouvertures 61 permet un débit élevé de liquide dans la conduite 11, et la fermeture hermétique du raccord 10 par le bouchon 66 permet d'éliminer toute fermeture supplémentaire dans les conduites 11 non utilisées du conduit 12.

La présence d'un trajet en labyrinthe reliant l'extérieur du corps de support 16 et du bouchon 38 avec la membrane 15 permet, d'une part, la pénétration de la vapeur pendant la stérilisation, mais empêche la pénétration de bactéries, de germes et d'organismes pathogènes, ce qui garantit la stérilité, même dans le cas d'une période de stockage prolongée. Enfin, les corps de support 16 et 49 des deux raccords 8 et 10 peuvent être réalisés à partir d'un matériau susceptible d'être stérilisé à une température élevée, jusqu'à 120°C, tout en restant facilement soudable par rapport, respectivement, au matériau de la poche 9 et à celui du tuyau 11, tout en possédant en outre les caractéristiques nécessaires relatives à l'utilisation médicale prévue.

Il est entendu que différents perfectionnements et modifications peuvent être apportés au dispositif de raccordement décrit sans que l'on ne s'écarte du cadre de l'invention. Par exemple, le raccord 8 peut être agencé avec un corps tubulaire 13 réalisé monobloc avec le corps de support 16. En outre, les ergots 45 et 74 peuvent être remplacés par des filetages complémentaires des filetages 18 et 53. On peut enfin modifier la forme et/ou le matériau des éléments individuels de chaque raccord 8, 10.

## Revendications

1. Dispositif (1) de raccordement entre un récipient et une conduite de passage pour liquides à usage médical, comprenant une membrane (15) et un élément perforateur (56) relié à une conduite (11) et à un récipient (9), caractérisé en ce que ladite membrane (15) et ledit élément perforateur (56) sont supportés, respectivement, par un premier et un deuxième raccord (8, 10), lesdits raccords étant pourvus de moyens de raccordement réciproque (18, 53), solidaires univoquement entre eux, pour le raccordement stable dudit récipient et de ladite conduite.

2. Dispositif selon la revendication 1, caractérisé en ce que lesdits moyens de raccordement réciproque définissent des moyens de vissage (18, 53), solidaires réciproquement.

3. Dispositif selon la revendication 2, caractérisé en ce que ledit élément perforateur (56) présente des moyens de perforation (58) pour la perforation de ladite membrane (15) au cours de l'étape de vissage desdits raccords (8, 10).

4. Dispositif selon la revendication 2 ou 3, caractérisé en ce que chacun desdits raccords (8, 10) comprend un corps de support (16, 49) normalement fermé par un bouchon correspondant (38, 66) doté de ses propres moyens de vissage (45, 74) solidaires desdits moyens de vissage (18, 53) du corps de support correspondant (16, 49).

5. Dispositif selon la revendication 4, caractérisé en ce que lesdits corps de support (16, 49) sont pourvus d'appendices ou d'ergots de prise (24, 54).

6. Dispositif selon la revendication 4 ou 5, caractérisé en ce que ledit corps de support (16) du premier raccord (8) comprend un manchon (17) pourvu de moyens de vissage (18), une partie tubulaire (19) agencée pour être fixée audit récipient (9), et une partie intermédiaire (21) entre ledit manchon (17) et ladite partie tubulaire (19), ladite partie intermédiaire étant pourvue de moyens d'engagement (26, 27, 36) en prise avec ladite membrane.

7. Dispositif selon la revendication 6, caractérisé en ce que ladite membrane (15) est réalisée monobloc avec un corps tubulaire (13) en matériau élastomère dilatable, ledit corps tubulaire étant pourvu de moyens d'engagement respectifs (29, 34) avec ledit corps de support (16) dudit premier raccord (8).

8. Dispositif selon la revendication 7, caractérisé en ce que ledit corps tubulaire (13) comporte au moins deux appendices ou ergots (34) enclenchés par déclic dans des fentes correspondantes (36) de ladite partie intermédiaire (21).

9. Dispositif selon la revendication 7 ou 8, caractérisé en ce que ladite partie intermédiaire (21) comprend une paire de rainures annulaires (26, 27), coaxiales entre elles, pour recevoir deux parties cylindriques correspondantes (29, 33) dudit corps tubulaire (13), respectivement intérieure et extérieure, ladite partie cylindrique extérieure (33) comportant lesdits appendices ou ergots (34) dudit corps tubulaire comprenant, en outre, une partie cylindrique supplémentaire (31) présentant un diamètre intérieur d'une première valeur, et s'étendant coaxialement et à la suite de ladite partie cylindrique intérieure (29), ladite partie cylindrique supplémentaire (31) étant séparée de ladite partie cylindrique intérieure (29) de ladite membrane (13).

10. Dispositif selon l'une quelconque des revendications 7 à 9, dans lequel ledit récipient (9) est une poche en matériau plastique souple, caractérisé en ce que ledit corps tubulaire (13) et ladite membrane (15) sont réalisés en PVC souple et en ce que ledit corps de support (16) dudit premier raccord (8) est en matériau plastique sensiblement rigide, par exemple, du polycarbonate, susceptible d'être soudé sur ladite poche (9).

11. Dispositif selon la revendication 9 ou 10, caractérisé en ce que ledit bouchon (38) est pourvu d'un élément intérieur annulaire (42) de guidage et de centrage de ladite partie cylindrique supplémentaire (31) dudit corps tubulaire (13), ledit élément annulaire intérieur et ladite partie cylindrique supplémentaire définissant un trajet en labyrinthe à partir de l'extérieur dudit bouchon vers ladite membrane (15) pour empêcher la pénétration d'organismes pathogènes.

12. Dispositif selon l'une quelconque des revendications 4 à 11, caractérisé en ce que ledit élément perforateur (56) présente une forme tubulaire se terminant par une pointe (57), au moins une ouverture (61) étant prévue au voisinage de ladite pointe (57).

13. Dispositif selon la revendication 12, caractérisé en ce que ladite pointe (57) présente une forme sensiblement conique définie par deux parties convergentes (58) et par deux parties parallèles (59), ladite pointe (57) s'étendant le long d'un diamètre dudit élément perforateur (56) et se séparant, réciproquement, en deux ouvertures d'entrée (61).

14. Dispositif selon la revendication 12 ou 13, caractérisé en ce que ledit élément perforateur (56) est réalisé monobloc avec un manchon (52) présentant lesdits moyens de vissage (53) et entourant extérieurement ledit élément perforateur (56).

15. Dispositif selon la revendication 9 et l'une des revendications 12 à 14, caractérisé en ce que ledit élément perforateur comprend une première section tubulaire (56a) dont le diamètre extérieur présente une deuxième valeur supérieure à ladite première valeur, une section tubulaire finale (56b), dont le diamètre extérieur présente une troisième valeur inférieure à ladite première valeur, et une section tubulaire intermédiaire (56c), dont le diamètre extérieur présente une valeur variable, comprise entre ladite deuxième et ladite troisième valeur.

16. Dispositif selon la revendication 15, caractérisé en ce que ladite partie cylindrique supplémentaire (31) dudit corps tubulaire (13) et ledit élément perforateur (56) ont une longueur telle que ladite pointe (57) dudit élément perforateur vient en interférence avec ladite membrane (13) avant que le raccordement desdits premier et deuxième raccords (8, 10) soit achevé; et que la longueur de ladite partie cylindrique supplémentaire (31) dudit corps tubulaire (13) est telle qu'elle vient en interférence avec la zone de diamètre agrandi de ladite partie intermédiaire (56c) dudit élément perforateur (56) avant que ladite pointe (57) dudit élément perforateur (56) ne rencontre ladite membrane (13) au cours de l'étape de raccordement desdits premier et deuxième raccords (8, 10).

17. Dispositif selon la revendication 15 ou 16, caractérisé en ce que ledit bouchon (66) dudit deuxième raccord (10) comporte intérieurement un manchon (72) partant d'une paroi de fond (67) dudit bouchon et dont la longueur est telle qu'il entoure ledit élément perforateur (56) à l'état vissé sur ledit corps de support (69), le diamètre intérieur dudit manchon (72) étant compris entre lesdites deuxième et troisième valeurs et étant réalisé en matériau plastique dilatable, pour venir en prise de façon étanche sur ledit élément perforateur à l'état vissé sur ledit corps de support (69).
